Europäisches Patentamt

European Patent Office

Office européen des brevets

(11) Veröffentlichungsnummer: **0 173 757**
**A1**

(12) # EUROPÄISCHE PATENTANMELDUNG

(21) Anmeldenummer: **84110421.9**

(22) Anmeldetag: **01.09.84**

(51) Int. Cl.⁴: **G 21 F 3/02**

(43) Veröffentlichungstag der Anmeldung:
**12.03.86 Patentblatt 86/11**

(84) Benannte Vertragsstaaten:
**AT BE CH FR GB IT LI LU NL SE**

(71) Anmelder: **STB Strahlentechnische Bilddiagnostik**
**Heinz Fleck**
**Haidkamp 95**
**D-2080 Pinneberg(DE)**

(72) Erfinder: **Lohse, Karl-Heinz**
**Im Bans 20a**
**D-2080 Pinneberg(DE)**

(74) Vertreter: **Fleck, Thomas, Dr.Dipl.-Chem. et al,**
**Patentanwälte Raffay, Fleck & Partner Postfach 32 32 17**
**D-2000 Hamburg 13(DE)**

(54) **Schürzenartige Maske für röntgenologische Aufnahmen.**

(57) Die vorliegende Erfindung betrifft eine schürzenartige Maske für röntgenologische Aufnahmen mit durchdringender Strahlung. Es war bisher nicht möglich, direkt eine Röntgenaufnahme von Schädel, Hals und Thorax eines Patienten herzustellen, die vergeichbare radiographische Schwärzungen bzw. Flächenkontraste aufweist. Hierzu schlägt die Erfindung eine schürzenartige Maske vor, die strahlenabsorbierendes Material, insbesondere Bleifolien unterschiedlicher Querschnittsdicke, aufweist bzw. eine unterschiedliche Anzahl von Bleifolien in den genannten Bereichen bei der Aufnahme vorsieht, um so die Schwärzungen auszugleichen und die gewünschte Kontrastabstufung zu erzielen.

Abb. 2.

EP 0 173 757 A1

Croydon Printing Company Ltd

Die vorliegende Erfindung betrifft eine schürzenartige Maske für röntgenologische Aufnahmen mit durchdringender Strahlung, insbesondere Angiographien.

Aus dem Stand der Technik sind verschiedene Röntgenmasken und -schilder bekanntgeworden, so z.B. aus der DE-OS 31 04 913. Die dort beschriebene Gesichtsmaske mit Brustschild dient jedoch zum Schutz für am Röntgengeräten arbeitende Personen, als auch für zu bestrahlende Patienten gegen Sekundär- und Streustrahlung.

Angiographische Verfahren verwenden Kontrastmittel zum röntgenphotographischen Darstellen von lebenden Organgefäßen, insbesondere des Menschen, bei dem man von einem darzustellenden Gefäßbereich eine Leeraufnahme herstellt, ein Kontrastmittel in den Gefäßbereich einleitet, und nach kurzer Zeit eine Aufnahme desselben Gefäßbereiches herstellt und anschließend daran durch photographische Substraktion der beiden Aufnahmen ein Betrachtungsbild herstellt. Es hat sich jedoch nachteilig bei solchen photographischen Verfahren bemerkbar gemacht, daß die Flächenkontraste zwischen Schädel, Hals und Thorax bei der Röntgenaufnahme so große Unterschiede aufweisen, daß das Subtraktionsbild nicht ermöglicht, den Verlauf der Gefäße vom Hals in den Schädel deutlich erkennen zu können. Derartige unvorteilhafte radiographische Schwärzungsunterschiede hat man ebenfalls schon festgestellt bei gleichzeitiger Aufnahme von Schädel und Hand, die dann durch Einsatz eines Strahlenfilters beseitigt wurden (vgl. DE-OS 26 59 419).

Der vorliegenden Erfindung liegt deshalb die Aufgabe zugrunde, die oben erwähnten Flächenkontraste bzw. radiographischen Schwärzungen so auszugleichen, daß die gewünschte Kontrastabstufung erzielt wird, insbesondere bei der Angiographie den Verlauf der Gefäße vom Hals in den Schädel deutlich erkennen zu können.

Diese Aufgabe wird grundsätzlich und erfindungsgemäß durch die im Anspruch 1 gekennzeichnete schürzenartige Maske gelöst. Das strahlenabsorbierende Material wird dabei in die Maske derart eingebettet bzw. eingearbeitet, daß eine unterschiedliche Querschnittsdicke und somit eine unterschiedliche Absorption in den von der Maske bedeckten Bereichen erzielt wird.

Vorteilhafterweise besteht die Maske aus einer Kunststoffplatte, die den Thorax und den Hals bedeckt und an diese Körperteile angepaßt ist. Die Halterung erfolgt mit Schulterschlaufen am Patienten. Eingebettet in die Platte bzw. Maske sind dünne Bleifolien, die im Halsbereich eine größere Querschnittsdicke besitzen als im Brustkorbbereich. Auf diese Art und Weise ist sichergestellt, daß für die angiographische Aufnahme der Schädel exponiert ist, während die beiden anderen Körperbereiche nicht überexponiert, sondern vielmehr einer geringeren Strahlungsmenge ausgesetzt sind, so daß die entsprechende Röntgenaufnahme bezüglich der Flächenkontraste nivelliert wird.

Weitere Vorteile und Merkmale sind in den vorstehenden Unteransprüchen beschrieben. Im folgenden wird ein Ausführungsbeispiel anhand der Zeichnung näher erläutert.

Es zeigt:

         Abb. 1     eine Frontansicht einer erfindungsgemäßen schürzenartigen Maske, wie sie am Patienten angelegt ist und

         Abb. 2     eine Seitenansicht der in Abb. 1 gezeigten erfindungsgemäßen Maske, wie sie am Patienten angelegt ist.

In Abb. 1 ist die erfindungsgemäße schürzenartige Maske allgemein mit 10 bezeichnet. Sie weist einen Halsteil 1 auf, der etwas schmaler ist als der darunterliegende Thorax- bzw. Brustkorbteil 2. Mit 3 sind die hinter bzw. unter den Armen verlaufenden Schultergurte bezeichnet, die zusammen mit dem Taillenbefestigungsgurt 4 dafür sorgen, daß die schürzenartige Maske sicher und rutschfrei am Patienten befestigt wird, von dem die Röntgenaufnahme erstellt werden soll.

Aus der Seitenansicht der Abb. 2 wird der erfindungsgemäße Aufbau der schürzenartigen Maske besser deutlich. In dem mit 1 bezeichneten Halsteil liegt nämlich eine stärkere Bleifüllung als im Thoraxbereich 2 vor, um eine gleiche Grundschwärzung auf der Röntgenaufnahme für Halsregion wie für Thorax und Schädel zu erreichen, damit letztendlich alle Bereiche miteinander unmittelbar und gleichzeitig miteinander vergleichbar sind. In diesem Ausführungsbeispiel ist die stärkere Bleifüllung durch zwei Bleifolien im Halsteil 1 dargestellt, während nur eine Bleifolie im Brustbereich 2 vorgesehen ist. Die Anzahl und Stärke der Bleifolien ist in keiner Weise kritisch und läßt sich in einem gewünschten Rahmen durchaus verändern. Die Kunststoffhülle für die Bleifolien ist allgemein mit 6 bezeichnet; sie ist dem Brustkorbbereich angepaßt und biegsamer Natur. Im oberen Halsteilbereich 1 weist sie ein weiches Kunststoffkinnpolster 5 auf, das dem Patientenkomfort dienen soll. Am Patienten wird die erfindungsgemäße schürzenartige Maske 10 mittels der Schultergurte 3 sowie des Taillenbefestigungsgurtes 4 angebracht.

EUROPEAN PATENT ATTORNEYS

POSTFACH 32 32 17
D-2000 HAMBURG 13

DIPL.-ING.  VINCENZ v. RAFFAY
DIPL.-CHEM. DR. THOMAS FLECK
HAMBURG

DIPL.-CHEM. DR. HANS D. BOETERS
DIPL.-ING. ROBERT BAUER
MÜNCHEN

KANZLEI:
GEFFCKENSTRASSE 6
TELEFON: (040) 47 80 23
TELEGRAMME: PATFAY, HAMBURG
TELEX 2 164 631 paty d

**0173757**

STB Strahlentechnische Bilddiagnostik Heinz Fleck

Haidkamp 95

2080 Pinneberg

UNSERE AKTE:    5014/32

## Schürzenartige Maske für röntgenologische Aufnahmen

### Patentansprüche

1.    Schürzenartige Maske für röntgenologische Aufnahmen mit durchdringender Strahlung, insbesondere Angiografien, gekennzeichnet durch strahlenabsorbierendes Material unterschiedlicher Querschnittsdicke.

2.    Maske nach Anspruch 1, dadurch gekennzeichnet, daß die Maske aus Kunststoff und das strahlenabsorbierende Material aus Bleifolien gebildet sind.

3.    Maske nach Anspruch 1 oder 2, dadurch gekennzeichnet, daß das strahlenabsorbierende Material bzw. die Bleifolien im Halsbereich des aufzunehmenden Patienten eine größere Querschnittsdicke besitzen als im Thorax- bzw. Brustkorbbereich, oder daß mehrere Folien in diesem Bereich übereinanderliegend angeordnet sind.

4.    Maske nach Anspruch 3, gekennzeichnet durch einen mehrschichtigen Aufbau zur steuerbaren Absorption.

5.     Maske nach Anspruch 1 bis 4, dadurch gekennzeichnet, daß sie in der Kontur den entsprechenden
Teilen des menschlichen Körpers angepaßt und etwas
flexibel ist.

6.     Maske nach Anspruch 1 bis 5, gekennzeichnet durch
Schulterschlaufen zur Befestigung am menschlichen
Körper.

7.     Maske nach Anspruch 1 bis 6, gekennzeichnet durch
eine Kunststoffrolle am Kinnanschluß.

Beschreibung:

Abb. 1.

Abb. 2.

Europäisches
Patentamt

EUROPÄISCHER RECHERCHENBERICHT

0173757
Nummer der Anmeldung

EP 84 11 0421

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.3) |
|---|---|---|---|
| E,X | DE-A-3 307 725 (STB)<br>* Insgesamt * | 1-7 | G 21 F 3/02 |
| | --- | | |
| A | GB-A-2 068 711 (MOTI)<br>* Zusammenfassung * & DE - A - 3 104 913 (Kat. D) | 1 | |
| | --- | | |
| A | GB-A-2 086 210 (MOTI)<br>* Seite 2, Zeilen 111-115; Figur 6 * | 1 | |
| | --- | | |
| A | US-A-3 569 713 (VIA)<br>* Zusammenfassung; Figuren 3,4 * | 2,5 | |
| | --- | | |
| A | GB-A-1 603 655 (WARDRAY)<br>* Seite 2, Zeilen 8-18; Figur 2 * | 1,2,5 | RECHERCHIERTE SACHGEBIETE (Int. Cl.3) |
| | --- | | |
| A | US-A-3 233 248 (BUSHNELL)<br>* Figur 1 * | 1 | G 21 F 3/00<br>A 61 B 6/00 |
| | --- | | |
| A | US-A-3 678 233 (FAW)<br>* Figur 8 * | 4 | |
| | ----- | | |

Der vorliegende Recherchenbericht wurde für alle Patentansprüche erstellt

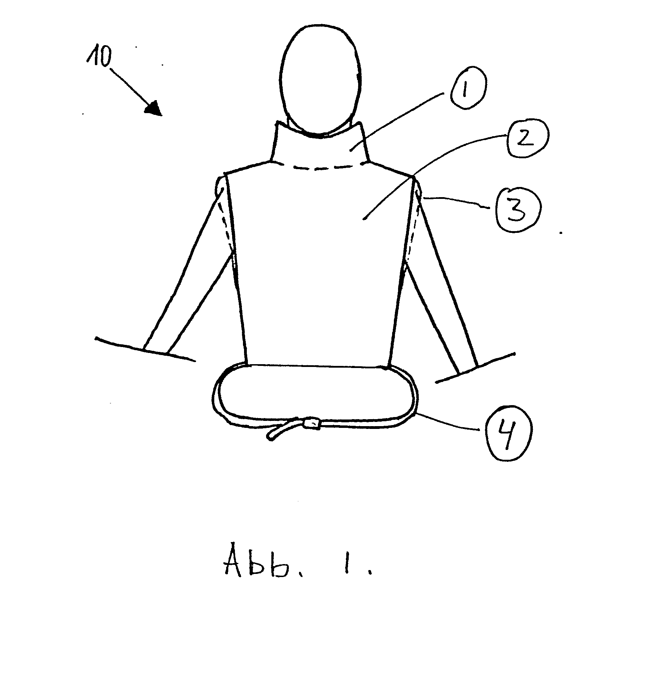

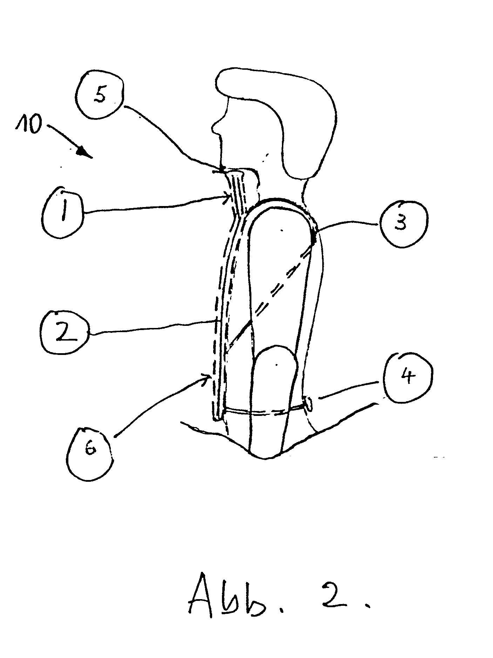

| Recherchenort<br>DEN HAAG | Abschlußdatum der Recherche<br>06-05-1985 | Prüfer<br>KAVCIC D. |
|---|---|---|